Europäisches Patentamt

European Patent Office

Office européen des brevets

(11) Veröffentlichungsnummer: **0 516 935 A2**

# EUROPÄISCHE PATENTANMELDUNG

(21) Anmeldenummer: **92105666.9**

(22) Anmeldetag: **02.04.92**

(51) Int. Cl.5: **C07C 63/16**, C07C 51/31, C07C 51/265

(30) Priorität: **04.06.91 DE 4118285**

(43) Veröffentlichungstag der Anmeldung: **09.12.92 Patentblatt 92/50**

(84) Benannte Vertragsstaaten: **BE DE FR GB**

(71) Anmelder: **HÜLS AKTIENGESELLSCHAFT Patentabteilung / PB 15 - Postfach 13 20 W-4370 Marl 1(DE)**

(72) Erfinder: **zur Hausen, Manfred, Dr. Hoher Weg 6 W-4235 Schermbeck(DE)**
Erfinder: **Kirste, Karl, Dr. Münsterlandstrasse 17 W-4370 Marl(DE)**
Erfinder: **Krix, Wilfried Ortbergstrasse 8 W-4250 Bottrop(DE)**

(54) **Verfahren zur weitgehend naphthochinonfreien Herstellung von Phthalsäureanhydrid aus Naphthalin.**

(57) Bei der katalytischen Luftoxidation von Naphthalin zu PSA entsteht als Nebenprodukt in erheblichen Mengen das Naphthochinon. Erfindungsgemäß läßt sich die Bildung dieses unerwünschten Nebenproduktes durch Einsatz eines weitgehend alkalifreien Naphthalins vermeiden.

EP 0 516 935 A2

Gegenstand der vorliegenden Erfindung ist ein Verfahren zur weitgehend naphthochinonfreien Herstellung von Phthalsäureanhydrid.

In neuerer Zeit ist man bei der Herstellung von Phthalsäureanhydrid (PSA) durch katalytische Luftoxidation von Naphthalin verstärkt daran interessiert, ein Produkt zu erhalten, das weitgehend naphthochinonfrei ist. Diese Forderung wird von Fachleuten mit dem Wunsch einer stetigen Qualitätsverbesserung, insbesondere einer möglichst niedrigen Farbzahl, begründet. Die strengen Auflagen im medizinischen Bereich haben dazu geführt, daß bereits geringe Naphthochinongehalte in den Weichmachern der für medizinische Zwecke verwendeten PVC-Materialien als bedenklich angesehen werden. Unabhängig hiervon werden erhöhte Naphthochinongehalte auch im Abgas von PSA-Anlagen als störend angesehen. Naphthochinon ist in der Bundesrepublik Deutschland als Stoff der Klasse I nach der "Technischen Anleitung zur Reinhaltung der Luft" (TA Luft) vom 27.02.86 eingestuft. Der zulässige Grenzwert liegt bei 20 mg pro $Nm^3$ Abgas.

Bei der Herstellung von PSA durch Luftoxidation von Naphthalin ist praktisch immer mit der Bildung von Naphthochinon zu rechnen (vgl. z. B. DE-OS 23 12 838).

In den letzten Jahren sind Katalysatoren entwickelt worden, die es gestatten, PSA mit einem Naphthochinongehalt von etwa 0,5 Gewichtsprozent Naphthochinon herzustellen (vgl. DE-OS 33 29 026).

Um einen Naphthochinongehalt von maximal 1 ppm zu erzielen, ist man gezwungen, das rohe PSA einer thermischen Behandlung in Gegenwart eines Katalysators oder eines Hilfsmittels zu unterziehen (vgl. z. B. DE-OSS 25 24 478, 26 54 405, 26 54 459, 33 29 026, 33 09 310, 35 02 682), wobei die Entfernung des Naphthochinons in der Regel durch Umwandlung in höher kondensierte Produkte erfolgt.

Die Nachteile dieser thermischen Behandlung lassen sich in 4 Punkten zusammenfassen:
1. Die sauren Kondensationskatalysatoren sind in der Regel stark korrosiv.
2. Die alkalischen Katalysatoren bewirken eine Decarboxylierung des PSA.
3. Die Verwendung spezieller ungesättigter Verbindungen, beispielsweise 1,5,9-Cyclododekatrien (vgl. DE-OS 35 02 682), ist aufwendig.
4. Die kondensierten Produkte erhöhen den bei der PSA-Destillation anfallenden Rückstand.

Es wäre wünschenswert, wenn es gelänge, selektivere Verfahren zur Herstellung von PSA zu entwickeln. Insbesondere sollte das rohe PSA weitgehend frei von Naphthochinon anfallen. Der gegenwärtige Stand der Technik liefert keine Anregungen, wie dies erreicht werden kann.

Es wurde jetzt überraschend gefunden, daß man bei der katalytischen Luftoxidation von Naphthalin ein weitgehend naphthochinonfreies PSA erhält, und daß auch das Abgas erheblich geringere Mengen an Naphthochinon enthält, wenn man von einem Naphthalin mit einem möglichst geringen Alkaligehalt ausgeht.

Die üblichen technischen Verfahren zur Herstellung von Naphthalin aus Steinkohleteer beinhalten eine Extraktion mit Natronlauge zur Entfernung von Phenolen (vgl. Ullmanns Encyclopädie der technischen Chemie, 4. Auflage, Band 17, Verlag Chemie, Weinheim 1979). Es verwundert daher nicht, daß das übliche im Handel erhältliche, großtechnisch hergestellte Naphthalin mehr als 20 ppm, vielfach sogar mehr als 200 ppm, Natrium enthält.

Die vorliegende Erfindung bezieht sich auf die Oxidation von Naphthalin in der Gasphase, wobei der Sauerstoff der Luft das Oxidationsmittel darstellt. Die Erfindung bezieht sich aber auch auf die sogenannte Mischoxidation, d. h. auf Verfahren, bei denen ein Gemisch aus Naphthalin und o-Xylol eingesetzt wird. Der Xylolanteil kann, bezogen auf das Gesamtgewicht beider Komponenten, bis zu 90 % betragen, vorzugsweise liegt er bei 40-60 %.

Als Katalysatoren eignen sich grundsätzlich alle zu diesem Zweck bekannten Katalysatoren, insbesondere die Katalysatoren auf Basis von Vanadiumpentoxid und Titandioxid sowie weiteren Zusätzen. Übliche Katalysatoren werden in der Fachliteratur beschrieben.

Es sind verschiedene Techniken bekannt, um Gemische aus Luft und dem/den aromatischen Kohlenwasserstoff/Kohlenwasserstoffen zu erzeugen. Für die vorliegende Erfindung ist die Art der Erzeugung dieser Gemische unkritisch.

Erfindungsgemäß ist es vorteilhaft, ein Naphthalin mit einem möglichst geringen Alkaligehalt einzusetzen. Vorzugsweise sollte dieser Gehalt unter 5 ppm, inbesondere unter 1 ppm, liegen. Aufgrund der technischen Herstellungsweise enthält das übliche Naphthalin Beimengungen an Alkalimetallen, insbesondere Natrium.

Gegenstand der Erfindung ist ein Verfahren zur weitgehend naphthochinonfreien Herstellung von PSA durch katalytische Luftoxidation von Naphthalin oder Gemischen aus Naphthalin und o-Xylol, welches dadurch gekennzeichnet ist, daß man ein Naphthalin einsetzt, das einen möglichst geringen Alkaligehalt aufweist.

Es sind dem Fachmann verschiedene Verfahren zur Abtrennung von Alkalimetallen aus Naphthalin bekannt, beispielsweise durch destillative Abtrennung.

Es hat sich als besonders günstig erwiesen, den bei der Luftoxidation verwendeten Katalysator ausschließlich mit praktisch alkalifreiem Naphthalin zu beschicken.

Für den Fachmann stellte das Nebenprodukt Naphthochinon bei der PSA-Herstellung bisher eine besondere Herausforderung dar, da es aufgrund seiner Wasserdampfflüchtigkeit außerordentlich schlecht abtrennbar ist. Nachteilig ist weiterhin, daß die bei der PSA-Destillation anfallenden Kondensationsprodukte auf Basis von Naphthochinon entsorgt werden müssen.

Im gleichen Maße, wie es erfindungsgemäß gelungen ist, den Naphthochinongehalt im PSA zu verringern, ist der Anfall von Destillationsrückständen zurückgegangen.

Mindest ebenso bedeutsam ist die Reduzierung des Naphthochinongehaltes im Abgas. Nach der bereits zitierten TA Luft ist die Emission eines Abgasstromes mit einem Gehalt von mehr als 20 mg Naphthochinon pro $Nm^3$ nicht zulässig. Dies bedeutet, daß solche Abgasströme nicht wie bisher einer Abgaswäsche unterworfen werden können, sondern einer thermischen oder katalytischen Verbrennung zugeführt werden müssen.

Erst die vorliegende Erfindung schafft die Voraussetzung für eine unproblematische Entsorgung des Abgases.

Neben den Vorteilen der höheren Selektivität ist die höhere Lebensdauer des Katalysators zu erwähnen. Während ein mit üblichem Naphthalin beschickter Katalysator bereits nach 12 bis 18 Monaten so hohe Naphthochinonmengen liefert, daß ein Weiterbetrieb unwirtschaftlich wird, kann ein Katalysator, der ausschließlich mit natriumfreiem Naphthalin behandelt wird, wesentlich länger betrieben werden.

**Experimenteller Teil**

Die Betriebsversuche wurden in einem Reaktor mit 8 168 Rohren mit einer Länge von 2,5 m und einem inneren Durchmesser von 25 mm durchgeführt. Die Rohre waren bis zu einer Füllhöhe von 2,3 m mit einem ringförmigen Trägerkatalysator der Fa. Wacker-Chemie (Typ NXR, äußerer Durchmesser 7 mm, Höhe 7 mm; aktive Masse auf Basis von Vanadiumpentoxid und Titandioxid) gefüllt. Die Naphthalin-Aufgabe erfolgte nach dem klassischen Sättigerprinzip. Die Luftmenge, die über den Reaktor gefahren wurde, betrug 28 600 $Nm^3$ pro Stunde bei einem Druck vor Reaktor von 1,4 bar.
Die Beladung betrug 60 g/$Nm^3$.
Das aus dem Reaktor austretende Reaktionsgas wurde über einen Gaskühler, Switch-Kondensatoren und einen Wascher mit angeschlossenem Kamin ins Freie geleitet. Das flüssig abgeschiedene Rohprodukt wurde destillativ aufgearbeitet.

Vergleichsversuch A

Der beschriebene Reaktor, dessen Rohre mit dem Katalysator Typ NXR der Fa. Wacker gefüllt waren, wurde kontinuierlich mit einem Naphthalin (mittlerer Alkaligehalt: 20 bis 200 ppm Natrium) beschickt. Der Naphthochinongehalt wurde werktäglich bestimmt und der monatliche mittlere Durchschnittswert ermittelt. In Abhängigkeit von der Lebensdauer des Katalysators wurden folgende Naphthochinongehalte im Roh-PSA ermittelt:

| Lebensdauer des Katalysators in Monaten | Naphthochinongehalt im Roh-PSA |
|---|---|
| 1 | 0,6 |
| 3 | 1,86 |
| 5 | 1,66 |
| 7 | 1,76 |
| 9 | 2,47 |
| 11 | 2,51 |
| 13 | 2,74 |
| 15 | 2,78 |
| 17 | 3,00 |

Der Naphthochinongehalt im Abgas betrug bereits nach wenigen Wochen mehr als 25 mg/$Nm^3$ und erhöhte sich mit zunehmender Laufzeit des Katalysators.

Während der Laufzeit des Katalysators mußte in der PSA-Destillation der Zusatz an Soda von 0,04 auf 0,08 % und der Zusatz an ungesättigten Ölen von 0,03 auf 0,24 %, jeweils bezogen auf die Gewichtsmenge des erzeugten Roh-PSA, erhöht werden, um den Naphthochinongehalt im Rein-PSA unter 2 ppm zu halten.

Die Salzbadtemperatur mußte in der gleichen Zeit von 365 auf 384 °C angehoben werden.

Bezogen auf die erzeugte Menge Roh-PSA stieg die Menge des bei der PSA-Destillation anfallenden Rückstandes während der Laufzeit des Katalysators von 2,5 auf 7,2 Gewichtsprozent. Gleichzeitig fiel die Rohausbeute von 96,5 % auf 94 %, bezogen auf die Gewichtsmenge des eingesetzten Naphthalins; hierdurch bedingt war nach 17 Monaten ein wirtschaftlicher Betrieb mit dem Katalysator nicht mehr möglich. Es mußte die Naphthalinoxidation unterbrochen und ein Katalysatorwechsel vorgenommen werden, was einen mehrwöchigen Anlagenstillstand zur Folge hatte.

Versuch 1

Der Vergleichsversuch A wurde mit der Änderung wiederholt, daß anstelle von rohem Naphthalin ein destilliertes Naphthalin mit einem Natriumgehalt von maximal 1 ppm eingesetzt wurde.

Der Naphthochinongehalt im Roh-PSA stieg im Laufe von 18 Monaten von 0,01 auf 0,05 %. Die Menge an Soda und ungesättigten Ölen von 0,02 bzw. 0,03 % reichte aus, um den Naphthochinongehalt im Rein-PSA unter 1 ppm zu halten. Die Menge des Destillationsrückstandes betrug während der ersten 18 Monate der Katalysatorstandzeit nahezu unverändert 0,4 %, bezogen auf die erzeugte Menge Roh-PSA.

Die Temperatur des Salzbades mußte innerhalb von 18 Monaten von 365 auf 375 °C erhöht werden, um den gewünschten Umsatz zu halten.

Versuch 2

Wie Versuch 1, jedoch wurde anstelle von reinem Naphthalin ein Gemisch aus Naphthalin und 5 % o-Xylol eingesetzt. Die Ergebnisse unterscheiden sich nicht von denen des Versuchs 1.

**Patentansprüche**

1.  Verfahren zur weitgehend naphthochinonfreien Herstellung von PSA durch katalytische Luftoxidation von Naphthalin oder Gemischen aus Naphthalin und o-Xylol,
    dadurch gekennzeichnet,
    daß man ein Naphthalin einsetzt, das einen möglichst geringen Alkaligehalt aufweist.

2.  Verfahren nach Anspruch 1,
    dadurch gekennzeichnet,
    daß man ein Naphthalin mit einem Alkaligehalt von weniger als 5 ppm, vorzugsweise weniger als 1 ppm, einsetzt.

3.  Verfahren zur weitgehend naphthochinonfreien Herstellung von PSA,
    dadurch gekennzeichnet,
    daß man den bei der Luftoxidation von Naphthalin eingesetzten Katalysator ausschließlich mit einem weitgehend alkalifreien Naphthalin gemäß den Ansprüchen 1 bis 2 beschickt.

4